# EUROPÄISCHE PATENTSCHRIFT

(11) **EP 2 247 276 B1**
(45) Veröffentlichungstag und Bekanntmachung des Hinweises auf die Patenterteilung: **25.12.2019**
(21) Anmeldenummer: 09704438.2
(22) Anmeldetag: 26.01.2009
(51) Int. Cl.: A61K 8/362, A61K 8/46, A61K 8/67, A61K 8/73, A61K 8/97, A61Q 7/00

(54) **HAARWUCHSMITTEL**
HAIR GROWTH AGENT
AGENT DE CROISSANCE CAPILLAIRE

(30) Priorität: 24.01.2008 DE 102008005972
(43) Veröffentlichungstag der Anmeldung: 10.11.2010
(73) Patentinhaber: Mathieu, Irmgard, 52372 Kreuzau-Drove (DE); Metzler, Heribert, 82362 Weilheim (Oberbayern) (DE)
(72) Erfinder: MATHIEU, Irmgard, 52372 Kreuzau-Drove (DE); MINGERS, Andreas, NL-6463 AV Kerkrade (NL); MATHIEU, Michel, 52372 Kreuzau-Drove (DE)
(74) Vertreter: Reuther, Martin
(86) Internationale Anmeldenummer: PCT/DE2009/000091
(87) Internationale Veröffentlichungsnummer: WO 2009/092369

(56) Entgegenhaltungen:
- EP-A- 1 402 874
- DE-A1- 3 118 882
- DE-U1- 29 812 754
- FR-A- 2 854 066
- FR-M- 2 014
- JP-A- 2004 155 734
- DATABASE WPI Week 200681 Thomson Scientific, London, GB; AN 2006-792498 XP002534222 & JP 2006 282609 A (NIPPON TENNENBUTSU KENKYUSHO KK) 19. Oktober 2006 (2006-10-19)
- DATABASE WPI Week 200169 Thomson Scientific, London, GB; AN 2001-605161 XP002534223 & JP 2001 172137 A (MORIKAWA T) 26. Juni 2001 (2001-06-26)
- ANDREAS SCHIEBER: "Knoblauch" [Online] Mai 2004 (2004-05), ROEMPP , INTERNET , XP002534167 Gefunden im Internet: URL:http://www.roempp.com/prod/index1.html > das ganze Dokument
- ANDREAS SCHIEBER: "allicin" November 2003 (2003-11), ROEMPP , INTERNET , XP002534168 Gefunden im Internet: URL:http://www.roempp.com/prod/index1.html > das ganze Dokument
- FLORIAN CONRAD STINTZING: "Zitrone" April 2003 (2003-04), ROEMPP , INTERNET , XP002534169 Gefunden im Internet: URL:http://www.roempp.com/prod/index1.html > das ganze Dokument
- Heilen mit Knoblauch und Zitrone , 12 November 2004 (2004-11-12), Retrieved from the Internet: URL:https://web.archive.org/web/2008091608 5716/http://www.chefkoch.de/forum/2,22,114 169/Heilen-mit-Knoblauch-und-Zitrone.html [retrieved on 2014-06-23]
- "Cook the Book : lemon and Garlic Chicken", , Retrieved from the Internet: URL:https://web.archive.org/web/2008041014 5658/http://www.seriouseats.com/recipes/20 07/07/cook-the-book-lemon-and-garlic.html [retrieved on 2014-06-23]
- DATABASE WPI Week 201313 Thomson Scientific, London, GB; AN 2012-Q41723 & CN 102 641 353 A (HE C) 22 August 2012 (2012-08-22)

## Beschreibung

Die Erfindung betrifft die Verwendung einer Mischung als Haarwuchsmittel.

Hierbei ist eine Vielzahl an Haarwuchsmitteln auf dem Markt bekannt. Allen ist jedoch gemein, dass die Wirksamkeit von vielen Bevölkerungskreisen in Frage gestellt wird. Dieses gilt auch für Haarwuchsmittel, wie sie aus der FR 2 014, der DE 22 06 568 A, der DE 20 2004 002 945 U1, der DE 198 58 670 C2, der EP 0 640 333 A2 oder der US 2007/0065395 A1 bekannt sind.

Es ist Aufgabe vorliegender Erfindung, ein Haarwuchsmittel bereitzustellen, welches äußerst wirksam ist. Diese Aufgabe wird durch eine Verwendung mit den Merkmalen der unabhängigen Ansprüche gelöst.

So kann sich ein Haarwuchsmittel durch einen Anteil an Vitamin C, einen Anteil an Allicin oder dessen Abbau- oder Stoffwechselprodukte, einen Anteil an Zitronensäure und durch einen Anteil an pflanzlichen Polysacchariden auszeichnen. Es wird vermutet, dass insbesondere die Kombination von Vitamin C, Allicin oder dessen Abbau- oder Stoffwechselprodukte und Polysacchariden, besonders wenn letztere in Form von Pektinen, insbesondere von Protopektinen, und/oder in Form von Mukopolysacchariden, also Schleimstoffen, vorliegen, zu der hervorragenden Wirksamkeit beiträgt.

In diesem Zusammenhang versteht es sich, dass, auch wenn die Pektine lediglich in Form von pektinhaltigen Stoffen vorliegen, die entsprechenden Vorteile erzielt werden können.

Auch kann sich ein Haarwuchsmittel durch einen Anteil an Pflanzenbestandeilen der Gattung Allium oder deren Stoffwechsel- oder Abbauprodukte und durch einen Anteil an Zitrusfruchtbestandteilen auszeichnen.

In vorliegendem Zusammenhang bezeichnet der Begriff "Bestandteile" natürliche Bestandteile, Extrakte oder sonstige Teile der jeweiligen Pflanzen oder Pflanzenteile, wie beispielsweise Knollen oder Früchte entsprechender Pflanzen, die insbesondere auch ggf. durch ihre genetische Signatur noch nachgewiesen werden können. Insoweit versteht es sich, dass nicht notwendigerweise ganze Pflanzen oder Pflanzenteile jeweils zur Anwendung kommen müssen. So können beispielsweise auch lediglich Früchte, Wurzeln oder Knollen als Pflanzenteile der jeweiligen Pflanze bzw. auch nur der Bestandteile, wie Fruchtschalen, Fruchtfleisch oder von trockenen Schalen befreite Knollen, zur Anwendung kommen.

Insoweit geht vorliegende Erfindung davon aus, dass insbesondere die Summe der Bestandteile, die erfindungsgemäß für das Haarwuchsmittel zur Anwendung kommen, und ihre Mischungsverhältnisse untereinander die erfindungsgemäßen Vorteile bedingen. Andererseits ist davon auszugehen, dass unter diesen Bestandteilen auch Bestandteile zu finden sind, die in ihrer Wirkung nur untergeordnet sind. Ebenso ist nicht auszuschließen, dass durch weitere Experimente es gelingt, die erfindungsgemäßen Vorteile vorliegender Erfindung auch mit synthetischen Stoffen, die den natürlichen Bestandteilen entsprechen, nachzustellen.

Unter Bezug auf die zweite der oben genannten erfindungsgemäßen Lösungen wird davon ausgegangen, dass insbesondere die Polysaccharide durch die natürlichen Bestandteile der oben genannten Pflanzen, seien es die Zitrusfrüchte oder seien es die Pflanzen der Gattung Allium, in einem äußerst breiten und wirksamen Spektrum bereitgestellt werden können. Andererseits ist auch diesbezüglich denkbar, synthetische Ersatzstoffe einzusetzen.

Insofern ist es von Vorteil, wenn der Anteil an Allicin oder dessen Stoffwechsel- bzw. Abbauprodukte 10 mg, vorzugsweise 20 mg, in 100 ml des erfindungsgemäßen Haarwuchsmittels übersteigt. Insoweit liegt ein verhältnismäßig hoher Anteil an Bestandteilen der Gattung Allium, insbesondere beispielsweise an Knoblauch, vor. Derartig hohe Mengen können allerdings ohne Weiteres auch aus anderen Naturprodukten, wie beispielsweise Bärlauch oder anderen Zwiebelgewächsen, bereitgestellt werden. Hierbei ist zu berücksichtigen, dass ein wesentlicher Bestandteil der Pflanzen dieser Gattung Allicin ist, welches jedoch verhältnismäßig schnell u.a. zu Ajoenen dimerisiert, ohne dass das erfindungsgemäße Haarwuchsmittel seine Wirkung verliert. Hierbei wird davon ausgegangen, dass auch die Abbauprodukte des Allicins bzw. dessen Stoffwechselprodukte, wenn es mit Körperzellen in Kontakt kommt und mit diesen wechselwirkt, für die eigentliche Wirkung dieses Anteils an dem erfindungsgemäßen Haarwuchsmittel verantwortlich sind.

Auch sollte der Anteil an Vitamin C vorzugsweise 0,002 mg bzw. 0,004 mg in 100 ml des erfindungsgemäßen Haarwuchsmittels übersteigen, was letztlich sowohl durch separate Beigabe von Vitamin C oder aber durch Ausnutzung des natürlichen Vitamingehalts der Zitrusfrüchte im Wesentlichen, oder aber auch der Pflanzen der Gattung Allium geschehen kann. Hierbei wird davon ausgegangen, dass ein derartig hoher Gehalt an Vitamin C entsprechend den Haarwuchs fördert. Besonders kann der Anteil an Vitamin C 10 mg bzw. 20 mg je 100 ml Haarwuchsmittel übersteigen.

Die Zitronensäure wird, wie bereits vorstehend dargestellt, vorzugsweise über natürlich belassene Zitrusfrüchte, die ggf. sogar mit Schale dem Haarwuchsmittel aufgegeben werden, bereitgestellt. Einen besonders hohen Anteil an Vitamin C und Zitronensäure können hierbei Zitronen beisteuern. Andererseits versteht es sich, dass die Zitronensäure auch als Konzentrat bzw. in synthetisch hergestellter Form dem Haarwuchsmittel aufgegeben werden kann. Vorzugsweise beträgt der Anteil an Zitronensäure in 100 ml Haarwuchsmittel so viel, wie aus 10 mg Zitronen, vorzugsweise aus 20 mg Zitronen, beigesteuert werden können.

Vorzugsweise enthält das Haarwuchsmittel auch einen Anteil an schwefelhaltigen ätherischen Ölen, wodurch die antiseptische Wirkung des Haarwuchsmittels, insbesondere bei einer topischen Anwendung, wie sie für das erfindungsgemäße Haarwuchsmittel vorgeschlagen wird, erhöht wird. Auch enthält das Haarwuchsmittel vorzugsweise einen Anteil an Sexualhormonen, die, wie bereits hinlänglich aus dem Stand der Technik bekannt, den Haarwuchs fördern können. Sowohl die schwefelhaltigen ätherischen Öle als auch die Sexualhormone können in nennenswertem Maße auch auf natürliche Weise, beispielsweise durch Knoblauch, bereitgestellt werden.

Die Wirkung des Haarwuchsmittels wird vorzugsweise durch einen Anteil an Vitamin A, der vorzugsweise über 200 pg, vorzugsweise über 400 pg, in 100 ml des Haarwuchsmittels liegt, entsprechend erhöht. Insbesondere kann der Anteil an Vitamin A jedoch auch 0,5 ng bzw. 0,7 ng übersteigen und sogar 2 ng oder 3 ng erreichen und übersteigen.

Dementsprechend kann das erfindungsgemäße Haarwuchsmittel auch einen Anteil an Vitamin B1 aufweisen, der vorzugsweise 0,10 mg, vorzugsweise 0,12 mg, in 100 ml des Haarwuchsmittels übersteigt. Insbesondere kann der Anteil an Vitamin B1 auch im Milligrammbereich je 100 ml Haarwuchsmittel liegen

Ebenso kann das Haarwuchsmittel einen Anteil an Vitamin B2 aufweisen, der vorzugsweise 0,12 mg bzw. 0,13 mg in 100 ml des Haarwuchsmittels übersteigt. Je nach konkreter Umsetzung können auch Anteile an Vitamin B2 im Milligrammbereich je 100 ml Haarwuchsmittel zu finden sein.

Auch ein Anteil an Vitamin PP, insbesondere wenn dieser 0,003 mg, vorzugsweise 0,004 mg, je 100 ml des Haarwuchsmittels übersteigt, ist entsprechend vorteilhaft. Auch der Anteil an Vitamin PP kann, je nach konkreter Umsetzung, vorzugsweise im Milligrammbereich je 100 ml Haarwuchsmittel zu finden sein.

Die vorgenannten Vitamine können einzeln oder in Kombination vorliegen und entsprechend die Wirkung des erfindungsgemäßen Haarwuchsmittels erhöhen, wobei zum jetzigen Erkenntnisstand davon ausgegangen wird, dass gerade die von Zitrusfrüchten bzw. von Pflanzen der Gattung Allium bereitgestellten Mengen entsprechend ausgewogen sind, um zu hervorragenden Ergebnissen zu führen.

Ebenso ist es vorteilhaft, wenn das Haarwuchsmittel einen Anteil an Apfelsäure aufweist. Hierbei wird davon ausgegangen, dass schon sehr geringe Anteile an Apfelsäure, wie sie beispielsweise in Zitrusfrüchten zu finden sind, entsprechend ausreichen, um zu der hervorragenden Wirkung des erfindungsgemäßen Haarwuchsmittels ergänzend beizutragen.

Vorzugsweise weist das erfindungsgemäße Haarwuchsmittel einen Anteil von mindestens 5 % Kohlenhydraten, allerdings bevorzugt weniger als 10 % Kohlenhydraten, auf. Hierbei wird davon ausgegangen, dass insbesondere die Auftragbarkeit des Haarwuchsmittels auf der Kopfhaut und eine entsprechende Verträglichkeit durch die obere Mengenbegrenzung sichergestellt werden kann, während die Kohlenhydrate an sich der Stabilisierung und Homogenisierung des Haarwuchsmittels förderlich erscheinen.

Durch einen Anteil an Fruchtzucker, der sich ggf. auf natürliche Weise aus den bereitgestellten Pflanzen ergeben kann, kann der Nährstoffgehalt, der durch das erfindungsgemäße Haarwuchsmittel auch bei einem Einmassieren auf der Kopfhaut der Haut bzw. den Haarwurzeln zugeführt werden kann, und somit die Wirksamkeit des Haarwuchsmittels in vorteilhafter Weise erhöht werden.

Ebenso können auch Mineralsalze, wie insbesondere Calcium und/oder Natrium bzw. Kalium, dem Haarwuchs förderlich sein und dementsprechend in dem Haarwuchsmittel vorgesehen sein. Vorzugsweise liegt hierbei Kalium in Form von Kaliumnitrat vor. Selbiges gilt alternativ bzw. kumulativ in Bezug auf Spurenelemente, die dementsprechend ebenfalls anteilig in dem Haarwuchsmittel vorgesehen sein können.

Flavonoide wirken in der Natur als natürliche Anti-Aging-Stoffe. Insofern ist es entsprechend vorteilhaft, wenn das Haarwuchsmittel einen Anteil an Flavonoiden aufweist, die entsprechend ihre Wirkung an Haar und Kopfhaut entfalten können. Dementsprechend erweisen sich insbesondere Glykoside als vorteilhaft. Ebenso können Quercentin und Kampferöl vorteilhaft wirksam genutzt werden. Auch können alternativ insbesondere aber kumulativ Terpene mit ihren die Durchblutung fördernden Eigenschaften, insbesondere im Zusammenspiel mit komplexen ätherischen Ölen vorteilhaft in dem Haarwuchsmittel zur Anwendung kommen.

Das Haarwuchsmittel kann darüber hinaus einen Anteil an natürlichen Enzymen aufweisen, welche es in seiner Wirkung unterstützen können.

Für die vorgenannten Anteile des erfindungsgemäßen Haarwuchsmittels kann insbesondere Wasser als Trägerstoff genutzt werden, wobei es sich versteht, dass diesbezüglich auch das in den eingebrachten Pflanzen vorhandene Wasser entsprechend zum Einsatz kommen kann. Insoweit auf eine zwischenzeitliche Trocknung der jeweiligen Pflanzen verzichtet wird, kann sichergestellt werden, dass ein äußerst großer Anteil der natürlichen Inhaltsstoffe der verwendeten Pflanzen in dem Haarwuchsmittel verbleibt und somit für entsprechende Anwendungen zur Verfügung steht.

Insoweit vorstehend genaue Mengenangaben fehlen, verstehen sich diese Mengenangaben als Äquivalente der jeweiligen Bestandteile der eingebrachten Mengen an pflanzlichen Ausgangsstoffen, wenn das Haarwuchsmittel aus Zitronen und Knoblauch mit 10 mg ganzer Zitronen auf 100 ml Haarwuchsmittel und einem Äquivalent von Knoblauch oder dessen Abbau- oder Stoffwechselprodukte, so dass 10 mg Allicin oder dessen Abbau- oder Stoffwechselprodukte in diesen 100 ml Haarwuchsmittel vorliegen, hergestellt wird.

Es versteht sich, dass die oben genannten Einzelanteile des erfindungsgemäßen Haarwuchsmittels in den beschriebenen Mengen auf jede beliebe Weise miteinander gemischt werden können, wobei, wie bereits mehrfach herausgestellt, vorzugsweise unmittelbar Naturprodukte zur Anwendung kommen und das Haarwuchsmittel insbesondere auf natürlicher bzw. rein biologischer Basis hergestellt werden kann. Auf diese Weise kann eine natürliche Mischung, die anscheinend besonders gut verträglich und auch besonders gut den Haarwuchs fördert, bereitgestellt werden.

Dem Haarwuchsmittel kann Alkohol, insbesondere Ethanol, als Konservierungsmittel aufgegeben werden. Hierbei sollte der Alkoholanteil vorzugsweise über 0,01 % liegen, um eine ausreichende Konservierung gewährleisten zu können. Andererseits ist es vorteilhaft, wenn der Anteil an Alkohol 10 % nicht übersteigt, um die einzelnen Anteile des Haarwuchsmittels in ihrer stofflichen Integrität nicht zu gefährden und insbesondere auch keine kontraproduktiven Effekte bei der Anwendung zu generieren. Vorzugsweise kommt Ethanol zur Anwendung, da dieses an sich hervorragend verträglich ist und eine ausreichend konservierende Wirkung aufweist. Insbesondere komplexere Alkohole sind zwar nicht ausgeschlossen, können jedoch ggf. zu unerwünschten Nebeneffekten führen.

Je nach konkreter Umsetzung können auch andere Konservierungsmaßnahmen, beispielsweise eine Bestrahlung, insbesondere eine sehr kurzzeitige aber hochenergetische Bestrahlung, oder ein Erhitzen, zur Anwendung kommen. Hierbei ist insbesondere die Intensität bzw. die Dauer der Konservierungsmaßnahme auf die Anteile des erfindungsgemäßen Haarwuchsmittels abzustimmen, um keine Schädigung der Inhaltsstoffe des Haarwuchsmittels durch die Konservierungsmaßnahmen zu bedingen. Hierbei können ggf. geringere Schädigungen in Kauf genommen werden, solange die Wirksamkeit in ausreichendem Maße gegeben ist. Im Zweifel ist durch geeignete Versuche ein Kompromiss zu suchen.

Es versteht sich aber auch, dass bereits die Merkmale der Unteransprüche einzeln das erfindungsgemäße oder auch ein anderes Haarwuchsmittel vorteilhaft verbessern können.

Ein erfindungsgemäßes Haarwuchsmittel als Ausführungsbeispiel enthält vorzugsweise zwischen 20 und 60 mg Allicin bzw. dessen Abbauprodukte, welches durch Knoblauchzehen dem Haarwuchsmittel aufgegeben wird. In alternativen Ausführungsformen kann das Allicin auch durch Bärlauch oder andere Zwiebelgewächse bereitgestellt werden.

Die hier aufgeführten Mengenangaben verstehen sich jeweils auf 100 ml des Haarwuchsmittels, wobei die Zitronensäure, neben Apfelsäure, bei diesem Ausführungsbeispiel im Wesentlichen aus Zitronen bereitgestellt ist. Bei diesem Ausführungsbeispiel sind je 100 ml Haarwuchsmittel 70 mg Zitronen eingearbeitet.

Der Anteil an Vitamin C liegt bei diesem Ausführungsbeispiel zwischen ungefähr 20 und 80 mg.

Auch weist das Haarwuchsmittel entsprechend seiner Ausgangspflanzenbestandteile einen Anteil an schwefelhaltigen ätherischen Ölen und/oder Sexualhormonen auf. Der Anteil an Vitamin A liegt zwischen 200 pg und 600 pg. Der Anteil an Vitamin B1 wird zwischen 0,5 mg und 3,0 mg gewählt. Er kann in einer alternativen Ausführungsform jedoch auch diese Werte übersteigen. Der Anteil an Vitamin B2 wird vorzugsweise zwischen 0,6 mg und 3,5 mg gewählt, wobei auch diese Werte in einer alternativen Ausführungsform überschritten werden dürfen. Darüber hinaus enthält dass Haarwuchsmittel zwischen 0,8 mg und 5,0 mg Vitamin PP, wobei auch diese Werte ggf. überschritten werden können.

Darüber hinaus enthält das Haarwuchsmittel nach diesem Ausführungsbeispiel Mineralsalze und Spurenelemente, wobei erstere insbesondere Calcium und Natrium sowie Kalium in Form von Kaliumnitrat betrifft. Auch enthält es Fruchtzucker und ungefähr 8 % Kohlenhydrate. Dazu kommen noch pektinhaltige Stoffe und Schleimstoffe als Polysaccharide sowie Glykoside als Lieferanten von Flavonoiden.

Im Übrigen enthält das Haarwuchsmittel nach diesem Ausführungsbeispiel als Trägerflüssigkeit im wesentlichen Wasser, wobei sich die vorgenannten Mengenangaben jeweils durchgängig auf 100 ml des Haarwuchsmittels beziehen.

Im Übrigen ist aus den vorgenannten Stoffangaben unmittelbar ersichtlich, dass das Haarwuchsmittel dieses Ausführungsbeispiels zur Gänze aus natürlich gewachsenen Produkten, insbesondere auf rein pflanzlicher Basis, hergestellt werden kann. Sämtliche Inhaltstoffe finden sich in pflanzlichen Produkten in ausreichender Menge und Konzentration.

Das Haarwuchsmittel sollte morgens und abends regelmäßig auf die Hautpartien, auf denen der Haarwuchs angeregt werden soll, aufgetragen werden. Bei allen bisherigen Probanden haben sich nach zwei bis drei Wochen die Haarfollikel geöffnet. Anschließend hat sich zunächst ein Haarflaum gebildet, der sich in den hierauf folgenden Wochen zu jungem Haar entwickelt. In der Regel haben nach ungefähr 4 Wochen die Haare angefangen zu sprießen, wobei es in Ausnahmefällen auch 7 bis 8 Wochen gedauert hat. Sämtliche Probanden berichteten von einem leicht prickelnden Gefühl nach dem Auftragen, was von den Probanden als subjektives Zeichen einer Reaktion gewertet wurde. Auch berichteten die Probanden, dass sich eine Schuppenbildung signifikant reduzierte.

## Patentansprüche

1. Nicht-therapeutische Verwendung einer Mischung aus einem Anteil an Vitamin C, einem Anteil an Allicin oder dessen Abbau- oder Stoffwechselprodukte, einem Anteil an Zitronensäure entsprechend mehr als 10 mg an ganzen Zitronen in 100 ml der Mischung, einem Anteil an pflanzlichen Polysacchariden sowie einem Anteil an Flavonoiden als Haarwuchsmittel zum Aufgetragen auf Hautpartien, auf denen der Haarwuchs angeregt werden soll, wobei diese Anteile der Mischung durch einen Anteil an Pflanzenbestandteilen der Gattung Allium oder deren Stoffwechsel- oder Abbauprodukte und durch einen Anteil von mehr als 10 mg an ganzen Zitronen in 100 ml der Mischung bereitgestellt ist.

2. Verwendung nach Anspruch 1, **dadurch gekennzeichnet, dass** die pflanzlichen Polysaccharide Pektine, insbesondere Protopektine, umfassen.

3. Verwendung nach Anspruch 2, **dadurch gekennzeichnet, dass** die Pektine in Form von pektinhaltigen Stoffen vorliegen.

4. Verwendung nach einem der vorstehenden Ansprüche, **dadurch gekennzeichnet, dass** die pflanzlichen Polysaccharide Mucopolysaccharide umfassen.

5. Verwendung nach einem der vorstehenden Ansprüche, **gekennzeichnet durch** einen Anteil an schwefelhaltigen ätherischen Ölen.

6. Verwendung nach einem der vorstehenden Ansprüche, **gekennzeichnet durch** einen Anteil an Sexualhormonen.

7. Verwendung nach einem der vorstehenden Ansprüche, **gekennzeichnet durch** einen Anteil an Zitronensäure entsprechend mehr als 20 mg Zitronen in 100 ml des Haarwuchsmittels.

8. Verwendung nach einem der vorstehenden Ansprüche, **gekennzeichnet durch** einen Anteil an Apfelsäure.

9. Verwendung nach einem der vorstehenden Ansprüche, **gekennzeichnet durch** einen Anteil an Flavonoid-Glykosiden.

10. Verwendung nach einem der vorstehenden Ansprüche, **gekennzeichnet durch** einen Anteil an Enzymen.

11. Verwendung nach einem der vorstehenden Ansprüche, **dadurch gekennzeichnet, dass** es auf natürlicher bzw. rein biologischer Basis hergestellt ist.

12. Verwendung nach einem der vorstehenden Ansprüche, **dadurch gekennzeichnet, dass** der Anteil an Pflanzenbestandteilen der Gattung Allium Knoblauchbestandteile umfasst.

13. Verwendung nach Anspruch 12, **dadurch gekennzeichnet, dass** der Anteil an Knoblauchbestandteile Knoblauch umfasst.

14. Verwendung nach einem der vorstehenden Ansprüche, **gekennzeichnet durch** einen Anteil an Alkohol, vorzugsweise an Ethanol.

## Claims

1. A non-therapeutic use of a mixture formed from a proportion of vitamin C, a proportion of allicin or degradation products or metabolites thereof, a proportion of citric acid corresponding to more than 10 mg of whole lemons in 100 mL of the mixture, a proportion of plant polysaccharides as well as a proportion of flavonoids, as a hair growth agent for application to parts of the skin on which hair growth is to be stimulated, wherein these proportions of the mixture are prepared via a proportion of plant components of the genus Allium or their metabolites or degradation products and via a proportion of more than 10 mg of whole lemons in 100 mL of the mixture.

2. The use as claimed in claim 1, **characterized in that** the plant polysaccharides comprise pectins, in particular protopectins.

3. The use as claimed in claim 2, **characterized in that** the pectins are present in the form of pectin-containing substances.

4. The use as claimed in one of the preceding claims, **characterized in that** the plant polysaccharides comprise mucopolysaccharides.

5. The use as claimed in one of the preceding claims, **characterized by** a proportion of sulphur-containing essential oils.

6. The use as claimed in one of the preceding claims, **characterized by** a proportion of sex hormones.

7. The use as claimed in one of the preceding claims, **characterized by** a proportion of citric acid corresponding to more than 20 mg of lemons in 100 mL of the hair growth agent.

8. The use as claimed in one of the preceding claims, **characterized by** a proportion of malic acid.

9. The use as claimed in one of the preceding claims, **characterized by** a proportion of flavonoid glycosides.

10. The use as claimed in one of the preceding claims, **characterized by** a proportion of enzymes.

11. The use as claimed in one of the preceding claims, **characterized in that** it is produced on a natural or purely biological basis.

12. The use as claimed in one of the preceding claims, **characterized in that** the proportion of plant components from the genus Allium comprises garlic components.

13. The use as claimed in claim 12, **characterized in that** the proportion of garlic components comprises garlic.

14. The use as claimed in one of the preceding claims, **characterized by** a proportion of alcohol, preferably ethanol.

## Revendications

1. Utilisation à des fins non thérapeutiques d'un mélange comprenant une part de vitamine C, une part d'allicine ou de ses produits de décomposition ou de ses produits métaboliques, une part d'acide citrique correspondant à plus de 10 mg de citrons entiers dans 100 ml du mélange, une part de polysaccharides végétaux, ainsi qu'une part de flavonoïdes comme agent de croissance capillaire destiné à être appliqué sur des parties cutanées sur lesquelles il s'agit de stimuler la croissance capillaire, lesdites parts du mélange étant mises à disposition par une part de composants végétaux du genre Allium ou de ses produits de décomposition ou de ses produits métaboliques et par une part de plus de 10 mg de citrons entiers dans 100 ml du mélange.

2. Utilisation selon la revendication 1, **caractérisée en ce que** les polysaccharides végétaux comprennent des pectines, notamment des protopectines.

3. Utilisation selon la revendication 2, **caractérisée en ce que** les pectines se présentent sous la forme de substances contenant des pectines.

4. Utilisation selon l'une quelconque des revendications précédentes, **caractérisée en ce que** les polysaccharides végétaux comprennent des mucopolysaccharides.

5. Utilisation selon l'une quelconque des revendications précédentes, **caractérisée par** une part d'huiles essentielles sulfureuses.

6. Utilisation selon l'une quelconque des revendications précédentes, **caractérisée par** une part d'hormones sexuelles.

7. Utilisation selon l'une quelconque des revendications précédentes, **caractérisée par** une part d'acide citrique, correspondant à plus de 20 mg de citrons dans 100 ml de l'agent de croissance capillaire.

8. Utilisation selon l'une quelconque des revendications précédentes, **caractérisée par** une part d'acide malique.

9. Utilisation selon l'une quelconque des revendications précédentes, **caractérisée par** une part de glycosides flavonoïdes.

10. Utilisation selon l'une quelconque des revendications précédentes, **caractérisée par** une part d'enzymes.

11. Utilisation selon l'une quelconque des revendications précédentes, **caractérisée en ce qu'**il est fabriqué sur base naturelle ou purement biologique.

12. Utilisation selon l'une quelconque des revendications précédentes, **caractérisée en ce que** la part de composants végétaux en comprend du genre Allium composants de l'ail.

13. Utilisation selon la revendication 12, **caractérisée en ce que** la part de composants de l'ail comprend de l'ail.

14. Utilisation selon l'une quelconque des revendications précédentes, **caractérisée par** une part d'alcool, notamment d'éthanol.
